# EUROPEAN PATENT SPECIFICATION

(11) **EP 3 669 824 B1**
(45) Date of publication and mention of the grant of the patent: **10.09.2025**
(21) Application number: 18215690.1
(22) Date of filing: 21.12.2018
(51) Int. Cl.: A61F 2/08, A61B 17/04

(54) **BONE IMPLANT**
KNOCHENIMPLANTAT
IMPLANT OSSEUX

(43) Date of publication of application: 24.06.2020
(73) Proprietor: Industrial Technology Research Institute, Chutung Hsinchu 310 (TW)
(72) Inventor: TSAI, Pei-I, Hsinchu City 300 (TW); SHEN, Hsin-Hsin, Taipei City 116 (TW); YANG, Kuo-Yi, Hsinchu City 300 (TW); HUANG, Chih-Chieh, Zhunan Township Miaoli Country 350 (TW); LIN, Shih-Ping, Kaohsiung City 805 (TW); LIN, De-Yau, Tainan City 722 (TW)
(74) Representative: Grünecker Patent- und Rechtsanwälte PartG mbB

(56) References cited:
- US-A1- 2006 276 895
- US-A1- 2009 319 043
- US-A1- 2015 250 513
- US-A1- 2016 100 870
- US-A1- 2016 166 301
- US-B2- 6 855 168

## Description

### [TECHNICAL FIELD]

The disclosure relates to a bone implant, more particularly a flexible bone implant.

### [BACKGROUND]

When human bones, tendons, ligaments or other human tissues are damaged to a certain extent, doctors may decide to operate a surgery for repair the damaged part, such as to relocate the fractured fragments onto their original positions and then to fix them in position with sutures and a bone implant (e.g., a bone screw) for them to self-repair, or to put an artificial implant in human body to replace the damaged part.

Fracture is common occurs in the shoulder, knee or other movable human portions, but the artificial implant is rigid and therefore to limit the movement of the portion it is implanted, which may make it difficult to move their fractured portion freely or may break the artificial implant as the movement exceeds the limitation. US 2009/0319043 A1 discloses a helicoil interference fixation system with a helical body comprising a plurality of turns separated by spaces therebetween. The inserter can be used for turning the helicoil, and the helicoil is mounted on the inserter in such a way that at least one strut of the helicoil is mounted in at least one groove of the inserter, such that rotation of the inserter causes rotation of the helicoil. US 2016/0100870 A1 discloses a bone implant with a body elongated along a longitudinal axis between a first end and a second end. The body comprises a rounded exterior surface portion and has a rounded-section transverse to the longitudinal axis. A tip is disposed proximal to a first end of the body and the head is disposed proximal to a second end of the body. A bore extends through at least a portion of the body and the window is disposed along the body and forming an opening. A thread winding is arranged around at least a portion of the rounded exterior surface portion. US 2016/0166301 A1 discloses a surgical implant comprising a body extending between an inner wall and an outer wall, wherein at least one porous region extends continuously around said body between said inner wall and said outer wall. The corresponding implant is empty along a longitudinal axis along which a cannulation is formed.

### [SUMMARY]

The invention is defined by independent claim 1. Preferred embodiments are included in the dependent claims.

Accordingly, the disclosure provides a bone implant which is more flexible and thus capable of decreasing the movement limitation to the injury portion and preventing the bone implant from being accidently detached.

One embodiment of the disclosure provides a bone implant. The bone implant includes at least one helix body and at least one pillar. The helix body surrounds an accommodating space. The pillar is disposed in the accommodating space and connected to the helix body. The pillar has at least two notches.

According to the bone implant as discussed above, the bone implant has the pillar to maintain the overall structural strength and has the notches to improve the flexibility. As such, the bone implants of the disclosure are able to decrease the movement limitation to the injury portion and prevent themselves from being detached.

The aforementioned summary and the following detailed description are set forth in order to provide a thorough understanding of the disclosed embodiment and provide a further explanations of claims of the disclosure.

### [BRIEF DESCRIPTION OF THE DRAWINGS]

FIG. 1 is a perspective view of a bone implant according to one embodiment of the disclosure;
FIG. 2 is a cross-sectional view of the bone implant in FIG. 1;
FIG. 3 is a side view of the bone implant in FIG. 1;
FIG. 4 is a perspective view of a bone implant according to another embodiment of disclosure;
FIG. 5 is a cross-sectional view of the bone implant in FIG. 4;
FIG. 6 is another cross-sectional view of the bone implant in FIG. 4;
FIG. 7 is a side view of the bone implant in FIG. 4;
FIG. 8 is a perspective view of a bone implant according to still another embodiment of the disclosure;
FIG. 9 is a perspective view of a bone implant according to yet another embodiment of the disclosure;
FIG. 10 is a cross-sectional view of the bone implant in FIG. 9;
FIG. 11 is another cross-sectional view of the bone implant in FIG. 9;
FIG. 12 is a perspective view of a bone implant according to still yet another embodiment of the disclosure;
FIG. 13 is a cross-sectional view of the bone implant in FIG. 12;
FIG. 14 is another cross-sectional view of the bone implant in FIG. 12;
FIG. 15 is a perspective view of a bone implant according to still yet further another embodiment of the disclosure;
FIG. 16 is a cross-sectional view of the bone implant in FIG. 15;
FIG. 17 is another cross-sectional view of the bone implant in FIG. 15;
FIG. 18 is a perspective view of a bone implant according to still a further embodiment of the disclosure;
FIG. 19 is a cross-sectional view of the bone implant in FIG. 18;
FIG. 20 is another cross-sectional view of the bone implant in FIG. 18;
FIG. 21 is a perspective view of a bone implant according to yet a further embodiment of the disclosure;
FIG. 22 is a cross-sectional view of the bone implant in FIG. 21;
FIG. 23 is another cross-sectional view of the bone implant in FIG. 21;
FIG. 24 is a perspective view of a bone implant according to still yet a further embodiment of the disclosure; and
FIG. 25 is a cross-sectional view of the bone implant in FIG. 24.

### [DETAILED DESCRIPTION]

In the following detailed description, for purposes of explanation, numerous specific details are set forth in order to provide a thorough understanding of the disclosed embodiments. According to detailed description, claims and figures, those having ordinary skill in the art can easily understand the purpose and the advantages of the disclosure and implement it. The invention is limited by the claims. In other instances, well-known structures and devices are schematically shown in order to simplify the drawing.

In the figures of the disclosure, the dimensions, ratios, angles, and so on may be exaggerated, but the present disclosure is not limited thereto. Various changes can be made without departing from the disclosure. The orientations mentioned in the description of the embodiments and the figures are for illustration, and the present disclosure is either not limited.

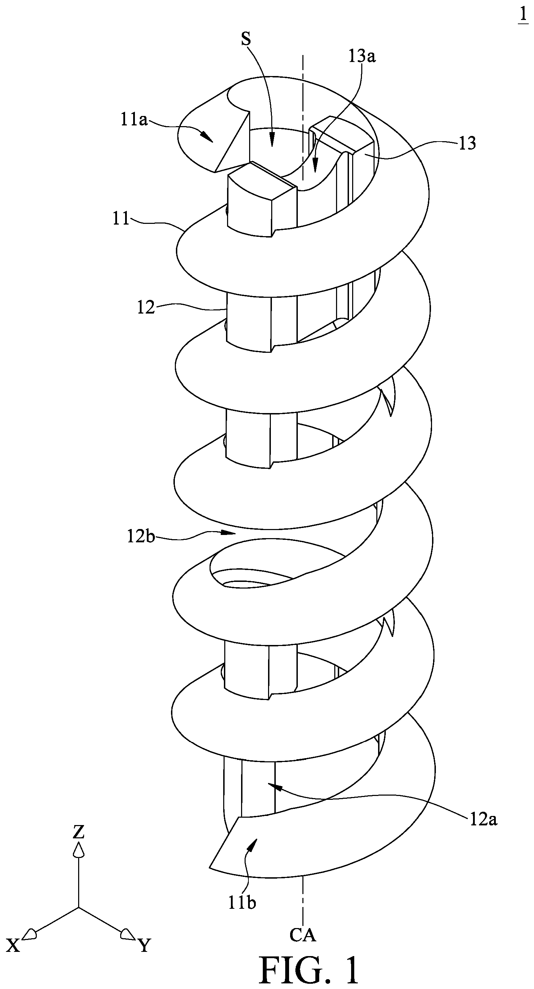

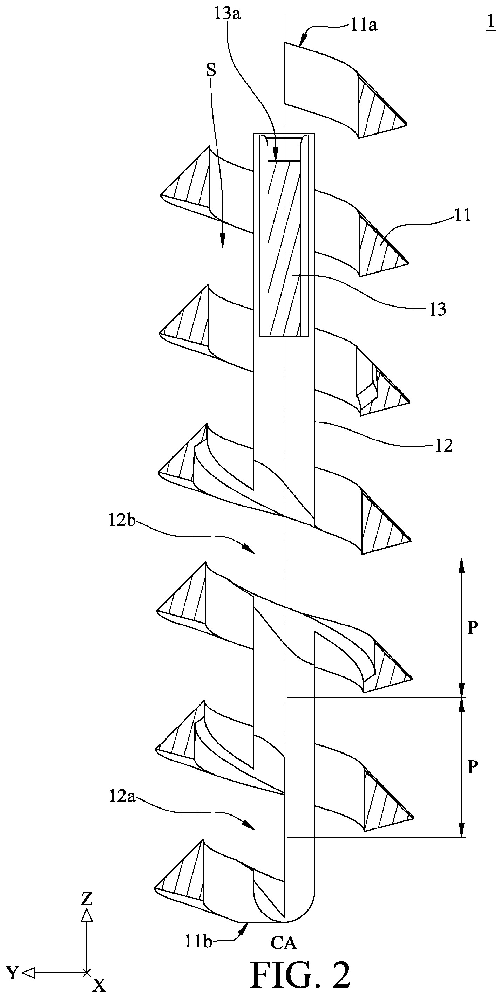

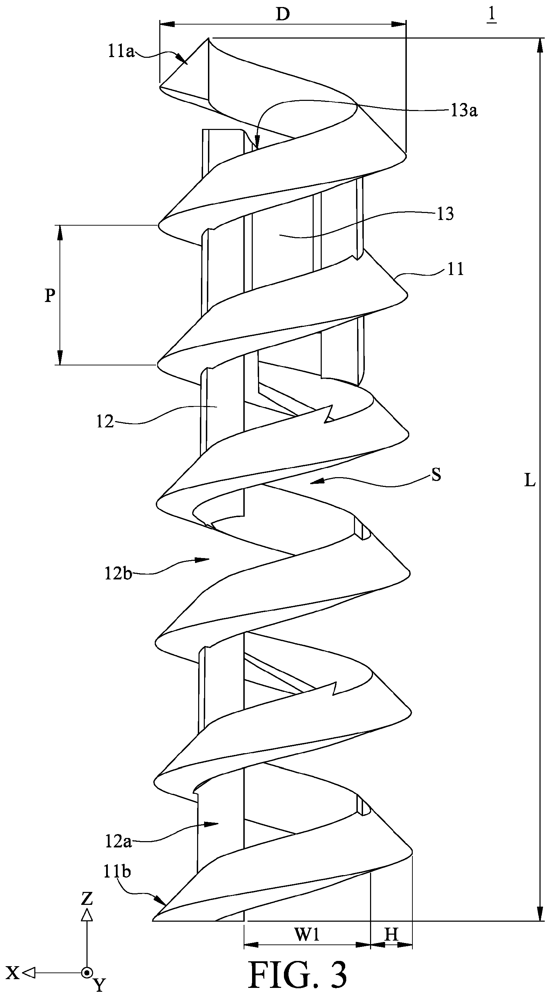

Please refer to FIG. 1 to FIG. 3. FIG. 1 is a perspective view of a bone implant according to one embodiment of the disclosure. FIG. 2 is a cross-sectional view of the bone implant in FIG. 1. FIG. 3 is a side view of the bone implant in FIG. 1.

This embodiment provides a bone implant 1. The bone implant 1 may include a helix body 11 and a pillar 12. The helix body 11 surrounds an accommodating space S. The pillar 12 is disposed in the accommodating space S and connected to the helix body 11. The pillar 12 has at least one notch.

In detail, the helix body 11 has two end portions 11a and 11b opposite to each other. The end portion 11a of the helix body 11 is configured to be implanted into human bone prior to the end portion 11b. The accommodating space S is surrounded by the helix body 11 about a central axis CA of the accommodating space S. In more detail, in this embodiment, the helix body 11 may consist of 6 turns, but the present disclosure is not limited thereto. In some other embodiments, the quantity of the turns of the helix body 11 may be modified in a range between 2 and 6 according to actual requirements. The helix body 11 has an outer diameter D, a length L and a thread depth H. In detail, the outer diameter D is a radial diameter of an outermost side of the helix body 11 passing through the central axis CA of the accommodating space S. The length L is an axial length of the helix body 11 measured in a direction parallel to the central axis CA of the accommodating space S. The thread depth H is the thickness of the solid portion of the helix body 11 in the direction perpendicular to the central axis CA of the accommodating space S. In this embodiment, the length L may be about 19.45 mm, the outer diameter D may be about 6 mm, and the thread depth H may be about 1.2 mm, but the present disclosure is not limited thereto. In some other embodiments, the outer diameter D and the length L may have a ratio between about 1 and 1/15. In addition, the helix body 11 is a right-handed helix structure, but the present disclosure is not limited thereto; in some other embodiments, the helix body may be a left-handed helix structure.

A pitch P is a distance between two adjacent crests of the helix body 11 in the direction parallel to the central axis CA. In this embodiment, each turn of the helix body 11 corresponds to a distance in the direction parallel to the central axis CA equal to the pitch P.

The pillar 12 is disposed in the accommodating space S and is inseparably connected to the helix body 11. The one or more notches on the pillar 12 make the pillar 12 partially narrower or divide the pillar 12 in parts. In the embodiment, the pillar 12 may have a narrowing type of notch 12a and a truncated type of notch 12b, and the narrowing type of notch 12a and the truncated type of notch 12b are located in an order from the end portion 11b to the end portion 11a of the helix body 11. The narrowing type of notch 12a makes the pillar 12 partially narrower, and the truncated type of notch 12b divides the pillar 12 in parts.

Further, in this embodiment, a distance between a center of the notch 12a and a center of the notch 12b may be equal to two times of the pitch P, but the present disclosure is not limited thereto. In some other embodiments, the pillar 12 may have at least one of the narrowing type of notch 12a and/or the truncated type of notch 12b. In detail, the pillar 12 may have the notch 12a and/or the notch 12b in every one to four pitches P. In the embodiment, the notch 12a and/or the notch 12b may be located closer to the end portion 11b than the end portion 11a, but the present disclosure is not limited thereto. In some other embodiments, the quantities and the arrangement of the narrowing type of notch 12a and the truncated type of notch 12b on the pillar 12 may be modified according to the required flexibility of the bone implant 1. The flexibility of the bone implant 1 may be increased due to the notch 12a and/or the notch 12b.

In this embodiment, the accommodating space S has a minimum width W1 between the pillar 12 and an inner side of the helix body 11 in the radial direction passing through the central axis CA. The minimum width W1 may be about 2.1 mm, but the present disclosure is not limited thereto. In some other embodiments, the minimum width W1 may be more than 0.9 mm according to the size of the tool (not shown in the figures) that is used to screw the bone implant 1 into the human bone. Note that the disclosure is not limited by the tool for screwing the bone implant.

In this embodiment, the bone implant 1 may further include a holding block 13 inseparably connected to the helix body 11 and the pillar 12. The holding block 13 is located inside the helix body 11 and in the end of the pillar 12. The holding block 13 may be located closer to the end portion 11a of the helix body 11 than the end portion 11b of the helix body 11. The holding block 13 has a recess 13a formed near the end portion 11a. The recess 13a is formed on a surface of the holding block 13 facing away from the end portion 11b of the helix body 11.

The bone implant 1 can be made by a 3D printer, a 3D printer and then being sintered, or a laser engraver.

A holding block 13 is adapted to fix a suture (not shown) in position on the bone implant 1. Before the bone implant 1 is implanted into a bone, the suture can be placed into the accommodating space S so as to be looped on the holding block 13 and located at the recess 13a of the holding block 13 so that the suture is fixed on the holding block 13. And then, the tool for screwing the bone implant 1 can be placed into the accommodating space S to clamp the pillar 12 without interfering with the suture. While the tool is being rotated about the central axis CA, the tool rotates the pillar 12 and the helix body 11 to rotate about the central axis CA as well, such that the bone implant 1 is able to be screwed into the bone by the helix body 11.

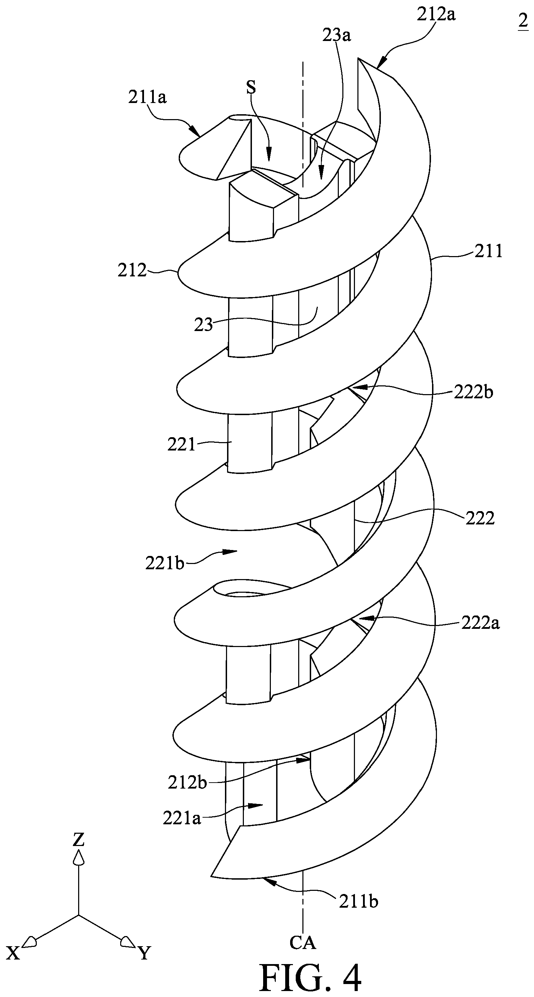

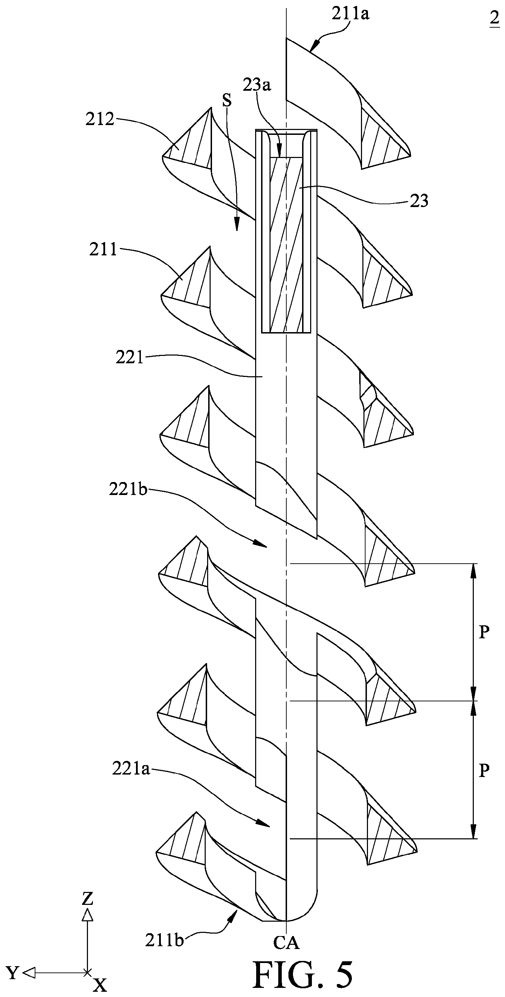

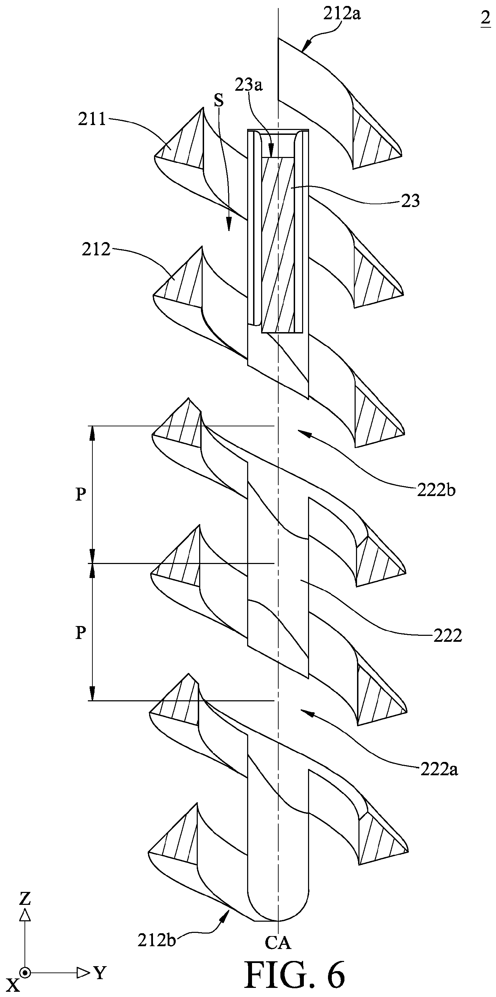

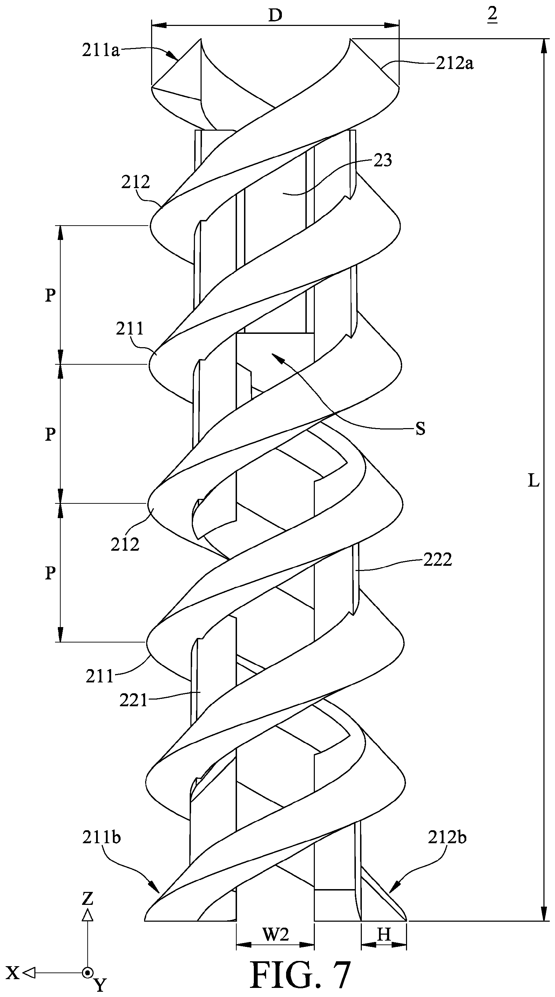

Then, please refer to FIG.4 to FIG. 7. FIG. 4 is a perspective view of a bone implant according to another embodiment of disclosure. FIG. 5 is a cross-sectional view of the bone implant in FIG. 4. FIG. 6 is another cross-sectional view of the bone implant in FIG. 4. FIG. 7 is a side view of the bone implant in FIG. 4.

This embodiment provides a bone implant 2. In this embodiment, the bone implant 2 may include two helix bodies 211 and 212, two pillars 221 and 222 and a holding block 23.

The helix body 211 has two end portions 211a and 211b opposite to each other. The end portion 211a is configured to be implanted into human bone prior to the end portion 211b. The helix body 211 surrounds the accommodating space S spirally about a central axis CA of the accommodating space S. In this embodiment, the helix body 211 may consist of 3 turns, but the present disclosure is not limited thereto. In some other embodiments, the quantity of the turns of the helix body 211 may be modified in a range between 2 to 6 according to actual requirements. In this embodiment, a length L of the helix body 211 may be about 19.45mm, an outer diameter D of the helix body 211 may be about 6mm, and a thread depth H of the helix body 211 may be about 1.2mm, but the present disclosure is not limited thereto. In some other embodiments, the outer diameter D and the length L of the helix body 211 may have a ratio between 1 and 1/15. In this embodiment, the helix body 211 is a right-handed helix structure, but the present disclosure is not limited thereto. In some other embodiments, the helix body may be a left-handed helix structure.

The helix body 212 is similar to the helix body 211. The helix body 212 has two end portion 212a and 212b opposite to each other. The end portion 212a is configured to be implanted into human bone prior to the end portion 212b. In this embodiment, the end portion 211a of the helix body 211 and the end portion 212a of the helix body 212 surround the accommodating space S from different positions and extend with a same direction respectively. In detail, the helix bodies 211 and 212 are radially offset with an angle of about 180 degrees from the central axis CA, and coaxial surround 3 turns about the central axis CA of the accommodating space S respectively. In this embodiment, the quantity of the helix bodies 211 and 212 is two, but it is not restricted. In some other embodiments, the quantity of the helix bodies may be two to four.

A distance between two adjacent turns of the helix bodies 211 and 212 in the direction parallel to the central axis CA is defined as a pitch P. In this embodiment, each turn of the helix body 211corresponds to a distance in the direction parallel to the central axis CA equal to two times of pitch P, and each turn of the helix body 212 corresponds to a distance in the direction parallel to the central axis CA equal to two times of pitch P.

The pillars 221 and 222 are located at different sides of the central axis CA. In this embodiment, the quantity of the pillars 221 and 222 is two, but it is not restricted.

The pillar 221 is disposed in the accommodating space S and is inseparably connected to the helix bodies 211 and 212. The pillar 221 may have a narrowing type of notch 221a and a truncated type of notch 221b, and the narrowing type of notch 221a and the truncated type of notch 221b are located in an order from the end portions 211b and 212b to the end portions 211a and 212a of the helix bodies 211 and 212.

In this embodiment, a distance between a center of the notch 221a and a center of the notch 221b may be equal to two times of pitch P, but the present disclosure is not limited thereto. In some other embodiments, the pillar 221 may have at least one of the narrowing type of notch 221a and/or the truncated type of notch 221b, and the pillar 221 may have the notch 221a and/or the notch 221b in every one to four pitches P.

The pillar 222 is disposed in the accommodating space S and is inseparably connected to the helix bodies 211 and 212. The pillar 222 may have two truncated type of notches 222a and 222b located in the order from the end portions 211b and 212b to the end portions 211a and 212a of the helix bodies 211 and 212.

In this embodiment, a distance between a center of the notch 222a and a center of the notch 222b may be equal to two times of pitch P, but the present disclosure is not limited thereto. In some other embodiments, the pillar 222 may have at least one of the narrowing type of notch and the truncated type of notch, and the pillar 222 may have the narrowing type of notch and/or the truncated type of notch in every one to four pitches P.

In this embodiment, the notches 221a and 221b of the pillar 221 and the notch 222a of the pillar 222 are located closer to the end portions 211b and 212b than the end portions 211a and 212a, and the notch 222b of the pillar 222 is located closer to the end portions 211a and 212a than the end portions 211b and 212b, but the present disclosure is not limited thereto. In some embodiments, the quantities and the arrangement of the notches on the pillars 221 and 222 may be modified according to the required flexibility of the bone implant 2. The flexibility of the bone implant 2 may be increased due to the narrowing type of notch and/or the truncated type of notch on the pillars 221 and/or 222.

**In** this embodiment, the accommodating space S has a minimum width W2 between the pillars 221 and 222 in a radial direction passing through the central axis CA, and the minimum width W2 may be about 2.1 mm, but the present disclosure is not limited thereto. **In** some other embodiments, the minimum width W2 may be more than 0.9 mm according to the size of the tool that is used to screw the bone implant 2 into the human bone.

The holding block 23 is located in the accommodating space S and is inseparably connected to the helix bodies 211 and 212 and the pillars 221 and 222. The holding block 23 is located inside the helix bodies 211 and 212 and located in the ends of the pillars 221 and 222. The holding block 23 is located closer to the end portions 211a and 212a than the end portions 211b and 212b. The holding block 23 has a recess 23a formed near the end portions 211a and 212a. The recess 23a is formed on a surface of the holding block 23 facing away from the end portions 211b and 212b of the helix bodies 211 and 212.

The bone implant 2 can be made by a 3D printing printer, a 3D printer and then being sintered or a laser engraver.

Before the bone implant 2 is implanted into bone, a suture can be placed into the accommodating space S so as to be looped on the holding block 23 and located at the recess 23a of the holding block 23 so that the suture is fixed on the holding block 23. And then, the tool for screwing the bone implant 2 is placed into the accommodating space S to clamp the pillars 221 and 222 without interfering with the suture. While the tool is being rotated about the central axis CA, the tool rotates the pillars 221 and 222 and the helix bodies 211 and 212 to rotate about the central axis CA as well, such that the bone implant 2 is able to be screwed into the bone by the helix bodies 211 and 212.

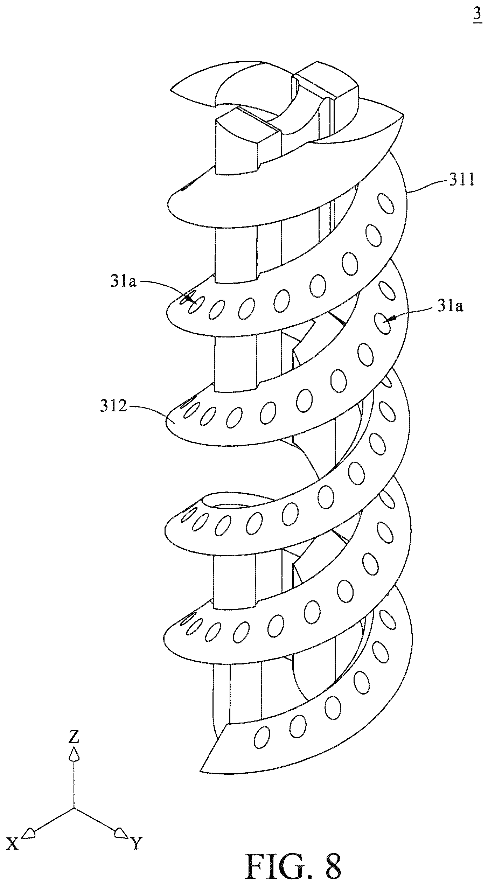

Please refer to FIG. 8. FIG. 8 is a perspective view of a bone implant according to still another embodiment of the disclosure. This embodiment provides a bone implant 3, and the bone implant 3 is similar to the bone implant 2 in FIG. 4.

It is noted that each of helix bodies 311 and 312 of the bone implant 3 has a plurality of holes 31a. After the bone implant 3 has been implanted into bone, osteoblast is allowed to grow bone in the holes 31a so as to improving bonding strength. In addition, in the case that the bone implant 3 is made of a degradable material, the holes 31a of the bone implant 3 can increase the contact areas between the bone and the bone implant 3, thereby improving the efficiency in degrading the bone implant 3.

The following introduces a plurality of bone implants similar to the bone implant 3 in FIG. 8 with unchanged parameters in the quantities of the pillars, the quantities of the notches, the pitch quantities of the distances between the notches, the quantities of the helix bodies, the outer diameters of the helix bodies, the lengths of the helix bodies, and the thread depths of the helix bodies. Compare with the maximum axial displacements of the bone implants in the central axis CA in different amount of turns. The larger displacement of the bone implant in the central axis CA, the greater flexibility. The results are shown in Table 1.

**≪Table 1≫**

| amount of turns | maximum axial displacement (mm) |
|---|---|
| 3 | 0.145 |
| 3.25 | 0.156 |
| 3.5 | 0.2629 |
| 3.75 | 0.3152 |
| 4 | 0.08418 |
| 4.25 | 0.09298 |

It can be seen that the bone implants with the helix bodies in 3.5 and 3.75 turns have larger maximum axial displacements than the others and thus having greater flexibility.

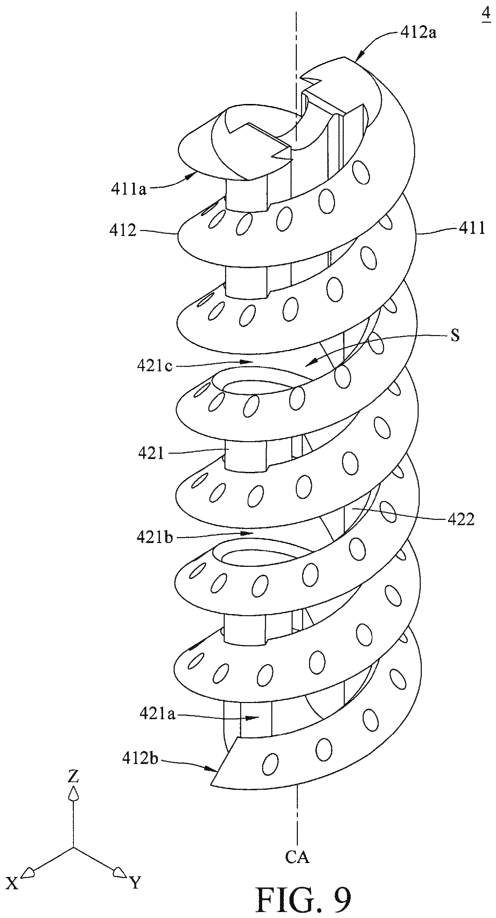

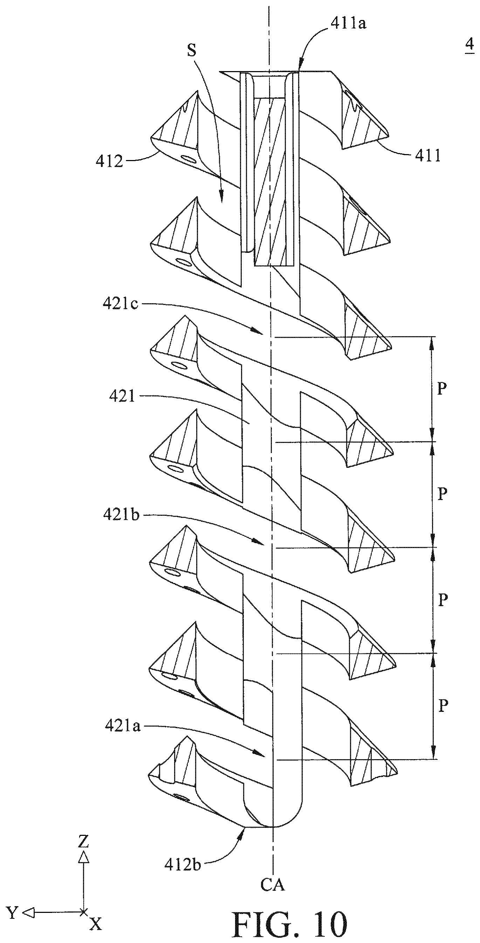

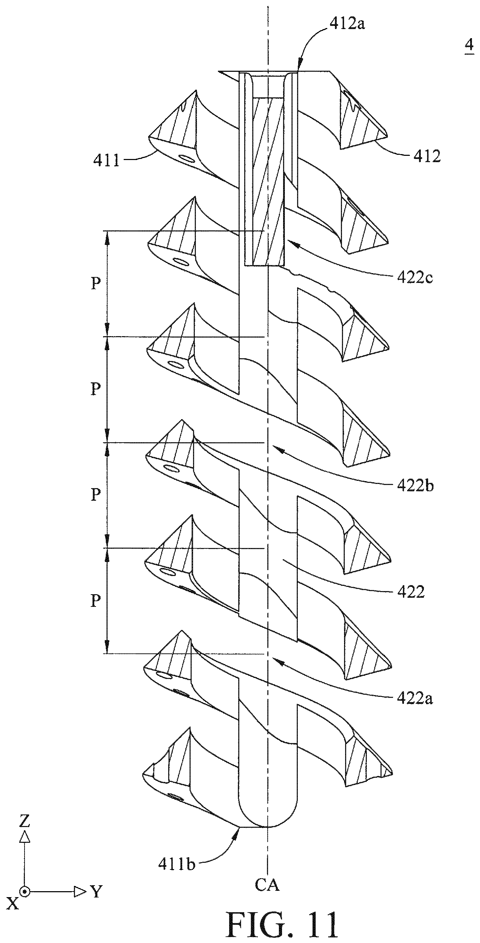

Please refer to FIG. 9 to FIG. 11. FIG. 9 is a perspective view of a bone implant according to yet another embodiment of the disclosure. FIG. 10 is a cross-sectional view of the bone implant in FIG. 9. FIG. 11 is another cross-sectional view of the bone implant in FIG. 9. This embodiment provides a bone implant 4, and the bone implant 4 is similar to the bone implant 3 in FIG. 8.

It is noted that, in this embodiment, the bone implant 4 has two helix bodies 411 and 412 surrounded an accommodating space S about a central axis CA. Each of the helix bodies 411 and 412 consists of 3.5 turns.

In this embodiment, the pillar 421 is disposed in the accommodating space S and is inseparably connected to the helix bodies 411 and 412. The pillar 421 may have a narrowing type of notch 421a and two truncated type of notches 421b and 421c, and the narrowing type of notch 421a and the truncated type of notches 421b and 421c are located in an order from the end portions 411b and 412b to the end portions 411a and 412a. A distance between a center of the notch 421a and a center of the notch 421b may be equal to two times of pitch P, and the distance between a center of the notch 421b and a center of the notch 421c may be equal to two times of pitch P, but the present disclosure is not limited thereto.

In this embodiment, the pillar 422 is disposed in the accommodating space S and is inseparably connected to the helix bodies 411 and 412. The pillar 422 may have two truncated type of notches 422a and 422b and a narrowing type of notch 422c, and the truncated type of notches 422a and 422b and the narrowing type of notch 422c are located in the order from the end portions 411b and 412b to the end portions 411a and 412a. A distance between a center of the notch 422a and a center of the notch 422b may be equal to two times of pitch P, and a distance between the center of the notch 422b and a center of the notch 422c may be equal to two times of pitch P, but the present disclosure is not limited thereto.

In this embodiment, the notches 421a and 421b of the pillar 421 and the notch 422a of the pillar 422 are located closer to the end portions 411b and 412b than the end portions 411a and 412a. The notch 422b is located close to center portions of the helix bodies 411 and 412. The notches 421c and 422c are located closer to the end portions 411a and 412a than the end portions 411b and 412b, but the present disclosure is not limited thereto. In other embodiments, the quantities and the arrangement of the notches on the pillars 421 and 422 may be modified according to the required flexibility of the bone implant 4. The flexibility of the bone implant 4 may be increased due to the narrowing type of notches and/or the truncated type of notches on the pillars 421 and/or 422.

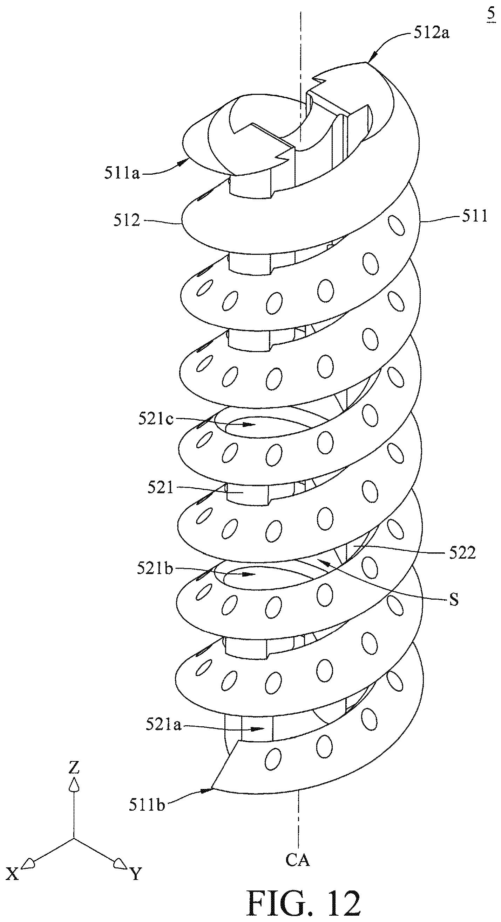

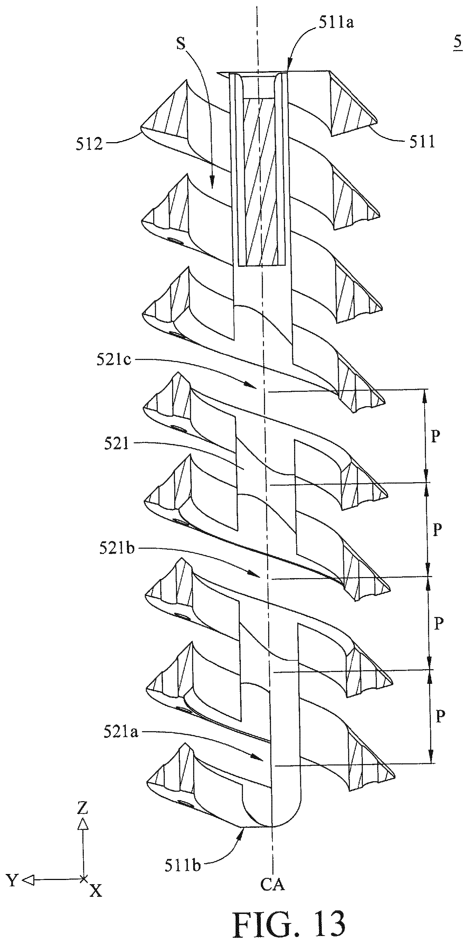

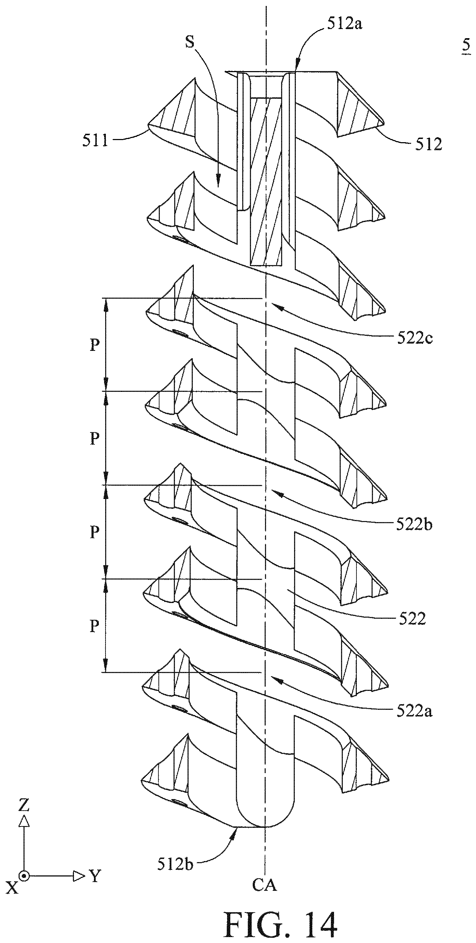

Please refer to FIG. 12 to FIG. 14. FIG. 12 is a perspective view of a bone implant according to still yet another embodiment of the disclosure. FIG. 13 is a cross-sectional view of the bone implant in FIG. 12. FIG. 14 is another cross-sectional view of the bone implant in FIG. 12. This embodiment provides a bone implant 5, and the bone implant 5 is similar to the bone implant 3 in FIG. 8.

It is noted that, in this embodiment, the bone implant 5 has two helix bodies 511 and 512 surrounded an accommodating space S about a central axis CA. Each of the helix bodies 511 and 512 consists of 4 turns.

In this embodiment, the pillar 521 is disposed in the accommodating space S and is inseparably connected to the helix bodies 511 and 512. The pillar 521 may have a narrowing type of notch 521a and two truncated type of notches 521b and 521c, and the narrowing type of notch 521a and the truncated type of notches 521b and 521c are located in an order from the end portions 511b and 512b to the end portions 511a and 512a. A distance between a center of the notch 521a and a center of the notch 521b may be equal to two times of pitch P, and a distance between the center of the notch 521b and a center of the notch 521c may be equal to two times of pitch P, but the present disclosure is not limited thereto.

**In** this embodiment, the pillar 522 is disposed in the accommodating space S and is inseparably connected to the helix bodies 511 and 512. The pillar 522 may have three truncated type of notches 522a, 522b and 522c, and the truncated type of notches 522a, 522b and 522c are located in the order from the end portions 511b and 512b to the end portions 511a and 512a. A distance between a center of the notch 522a and a center of the notch 522b may be equal to two times of pitch P, and a distance between the center of the notch 522b and a center of the notch 522c may be equal to two times of pitch P, but the present disclosure is not limited thereto.

In this embodiment, the notches 521a and 521b of the pillar 521 and the notches 522a and 522b of the pillar 522 are located closer to the end portions 511b and 512b than the end portions 511a and 512a. The notches 521c and 522c are located closer to the end portions 511a and 512a than the end portions 511b and 512b, but the present disclosure is not limited thereto. In other embodiments, the quantities and the arrangement of the notches on the pillars 521 and 522 may be modified according to the required flexibility of the bone implant 5. The flexibility of the bone implant 5 may be increased due to the narrowing type of notches and/or the truncated type of notches on the pillars 521 and/or 522.

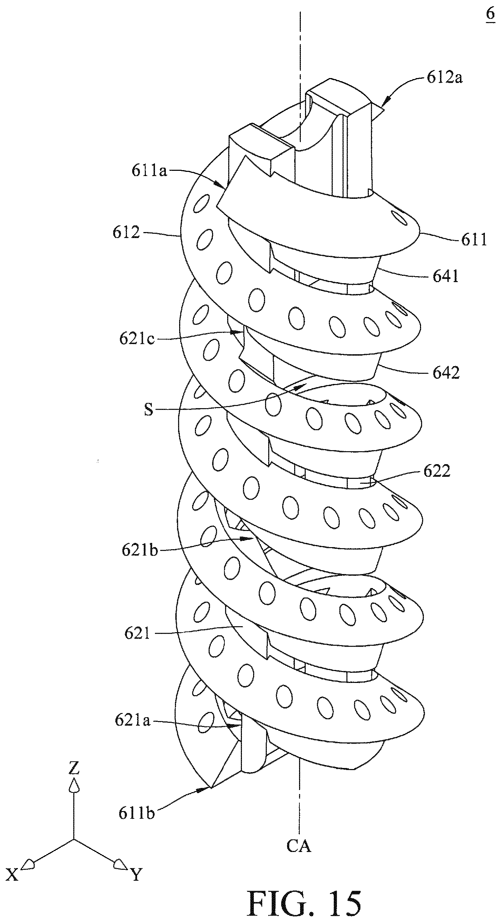

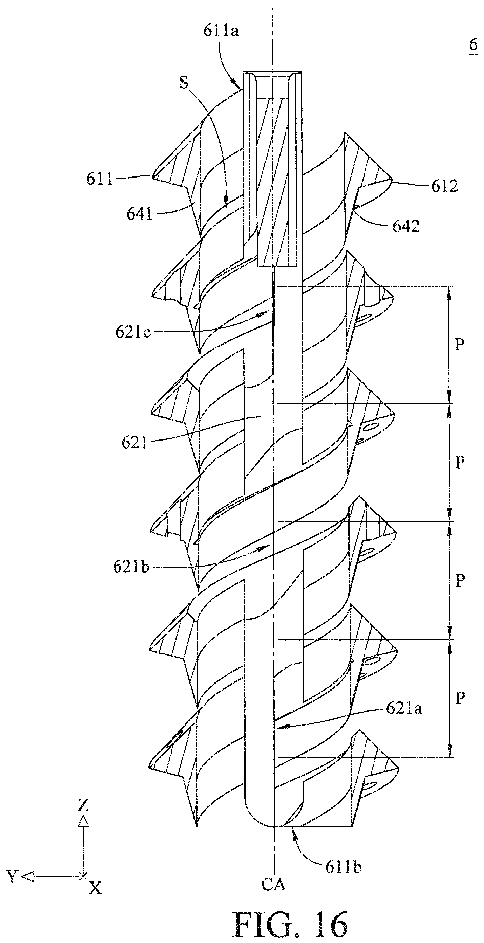

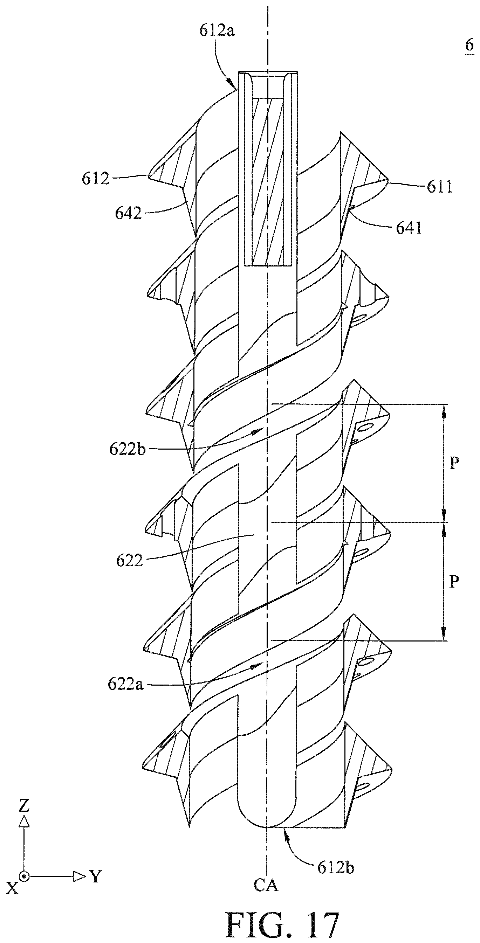

Then, please refer to FIG. 15 to FIG. 17. FIG. 15 is a perspective view of a bone implant according to still yet further another embodiment of the disclosure. FIG. 16 is a cross-sectional view of the bone implant in FIG. 15. FIG. 17 is another cross-sectional view of the bone implant in FIG. 15. This embodiment provides a bone implant 6, and the bone implant 6 is similar to the bone implant 3 in FIG. 8.

It is noted that, in this embodiment, the bone implant 6 has two helix bodies 611 and 612 surrounded an accommodating space S about a central axis CA. **In** this embodiment, each of the two helix bodies 611 and 612 is a left-handed helix structure, but the present disclosure is not limited thereto.

**In** this embodiment, the pillar 621 is disposed in the accommodating space S and is inseparably connected to the two helix bodies 611 and 612. The pillar 621 may have a narrowing type of notch 621a, a truncated type of notch 621b and a narrowing type of notch 621c located in an order from the end portions 611b and 612b to the end portions 611a and 612a. A distance between a center of the notch 621a and a center of the notch 621b may be equal to two times of pitch **P,** and a distance between the center of the notch 621b and a center of the notch 621c may be equal to two times of pitch **P,** but the present disclosure is not limited thereto.

In this embodiment, the pillar 622 is disposed in the accommodating space S and is inseparably connected to the helix bodies 611 and 612. The pillar 622 may have two truncated type of notches 622a and 622b located in the order from the end portions 611b and 612b to the end portions 611a and 612a. A distance between a center of the notch 621a and a center of the notch 421b may be equal to two times of pitch P, but the present disclosure is not limited thereto.

In this embodiment, the notches 621a and 621b of the pillar 621 and the notch 622a of the pillar 622 are located closer to the end portions 611b and 612b than the end portions 611a and 612a. The notches 621c and 622b are located closer to the end portions 611a and 612a than the end portions 611b and 612b, but the present disclosure is not limited thereto. In other embodiments, the quantities and the arrangement of the notches on the pillars 621 and/or 622 may be modified according to the required flexibility of the bone implant 6. The flexibility of the bone implant 6 may be increased due to the narrowing type of notches and/or the truncated type of notches on the pillars 621 and/or 622.

In this embodiment, the bone implant 6 may further include walls 641 and 642. The wall 641 is inseparably connected to the helix body 611 and surrounds the accommodating space S with the helix body 611. The wall 641 extends from the helix body 611 in the direction parallel to the central axis CA. The wall 642 is inseparably connected to the helix body 612 and surrounds the accommodating space S with the helix body 612. The wall 642 extends from the helix body 612 in the direction parallel to the central axis CA. When the bone implant 6 is implanted into bone, the walls 641 and 642 of the bone implant 6 can increase the contact area between the bone and the bone implant 6.

In this embodiment, the walls 641 and 642 has no hole, but the present disclosure is not limited thereto. In some other embodiments, each of the walls 641 and 642 may have a plurality of holes. In such a case, after the bone implant 6 has been implanted into bone, osteoblast is allowed to grow bone in the holes so as to improve bonding strength. In addition, in the case that the bone implant 6 is made of a degradable material, the holes of the wall 641 and 642 can increase the contact areas between the bone and the bone implant6, thereby improving the efficiency in degrading the bone implant 6.

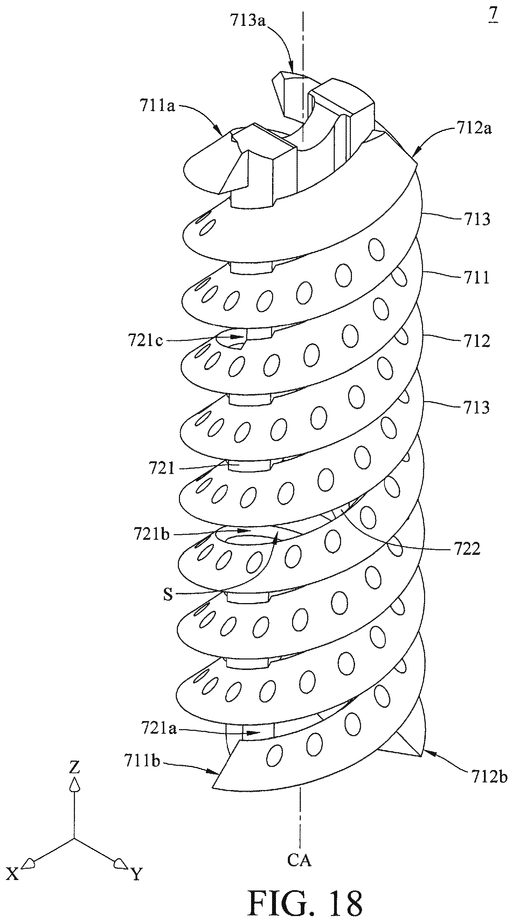

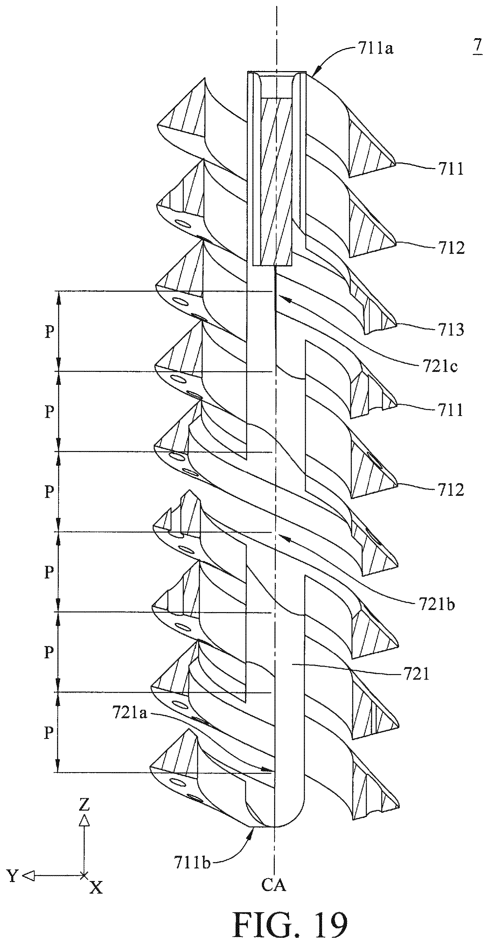

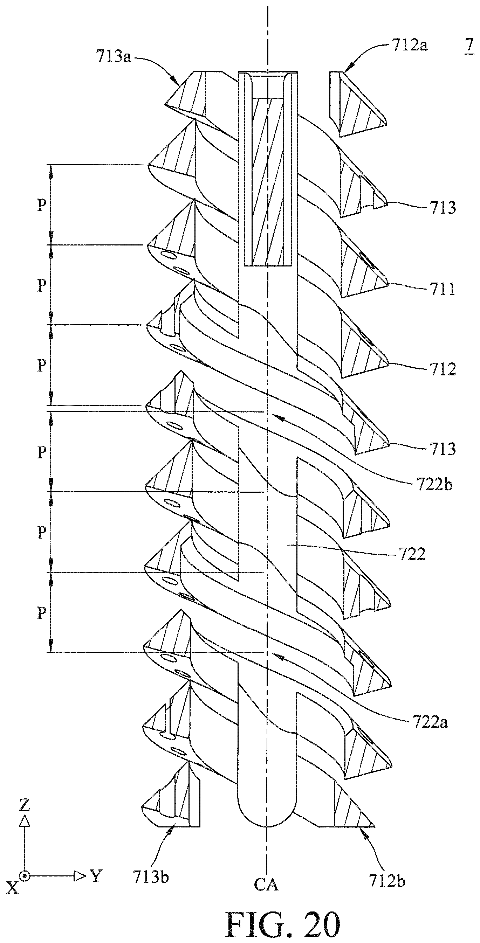

Please refer to FIG. 18 to FIG. 20. FIG. 18 is a perspective view of a bone implant according to still a further embodiment of the disclosure. FIG. 19 is a cross-sectional view of the bone implant in FIG. 18. FIG. 20 is another cross-sectional view of the bone implant in FIG. 18. This embodiment provides a bone implant 7, and the bone implant 7 is similar to the bone implant 3 in FIG. 8.

It is noted that in this embodiment, the bone implant 7 may have three helix bodies 711, 712 and 713. In this embodiment, the end portion 711a of the helix body 711, the end portion 712a of the helix body 712 and the end portion 713a of the helix body 713 surround the accommodating space S from different positions and extend with a same direction respectively. In detail, the helix bodies 711, 712 and 713 are radially offset with an angle difference of 120 degrees from the central axis CA, and coaxial surround 3 turns about the central axis CA of the accommodating space S respectively.

A distance between two adjacent turns of two helix bodies among the helix bodies 711, 712 and 713 in the direction parallel to the central axis CA is defined as a pitch P. In this embodiment, each turn of the helix body 711 corresponds to a distance in the direction parallel to the central axis CA equal to three times of pitch **P,** each turn of the helix body 712 corresponds to a distance in the direction parallel to the central axis CA equal to three times of pitch **P,** and each turn of the helix body 713 corresponds to a distance in the direction parallel to the central axis CA equal to three times of pitch P.

**In** this embodiment, the pillar 721 is disposed in the accommodating space S and is inseparably connected to the helix bodies 711, 712 and 713. The pillar 721 may have a narrowing type of notch 721a, a truncated type of notch 721b and a narrowing type of notch 721c located in an order from the end portions 711b, 712b and 713b to the end portions 711a, 712a and 713a. A distance between a center of the notch 721a and a center of the notch 721b may be equal to three times of pitch **P,** and a distance between the center of the notch 721b and a center of the notch 721c may be equal to three times of pitch P, but the present disclosure is not limited thereto.

In this embodiment, the pillar 722 is disposed in the accommodating space S and is inseparably connected to the helix bodies 711, 712 and 713. The pillar 722 may have two truncated type of notches 722a and 722b located in the order from the end portions 711b, 712b and 713b to the end portions 711a, 712a and 713a. A distance between a center of the notch 722a and a center of the notch 722b may be equal to three times of pitch P, but the present disclosure is not limited thereto.

In this embodiment, the notches 721a and 721b of the pillar 721 and the notch 722a of the pillar 722 are located closer to the end portions 711b, 712b and 713b than the end portions 711a, 712a and 713a. The notches 721c and 722b are located closer to the end portions 711a, 712a and 713a than the end portions 711b, 712b and 713b, but the present disclosure is not limited thereto. In other embodiments, the quantities and the arrangement of the notches on the pillars 721 and 722 may be modified according to the required flexibility of the bone implant 7. The flexibility of the bone implant 7 may be increased due to the narrowing type of notches and/or the truncated type of notches on the pillars 721 and/or 722.

The following introduces a plurality of bone implants similar to the bone implant 7 in FIG. 18 with unchanged parameters in the quantities of the pillars, the quantities of the notches, the pitch quantities of the distances between the notches, the quantities of the helix bodies, the outer diameters of the helix bodies, the lengths of the helix bodies, and the thread depths of the helix bodies. Compare with the maximum axial displacements of the bone implants in the central axis CA in different amount of turns. The larger displacement of the bone implant in the central axis CA, the greater flexibility. The results are shown in Table 2.

**≪Table 2≫**

| amount of turns | maximum axial displacement (mm) |
|---|---|
| 3 | 0.1147 |
| 3.25 | 0.1215 |
| 3.5 | 0.1211 |
| 3.75 | 0.04917 |

It can be seen that the bone implants with the helix bodies in 3, 3.25 and 3.5 turns have larger maximum axial displacements than the other and thus having greater flexibility.

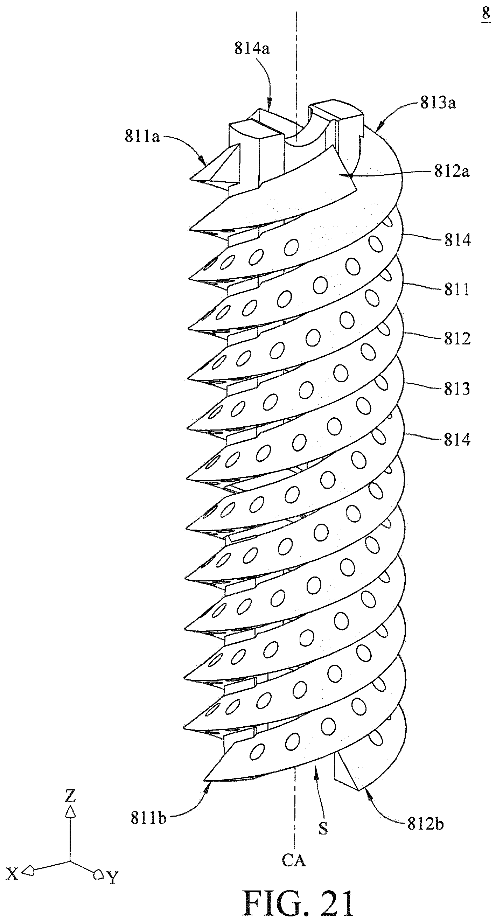

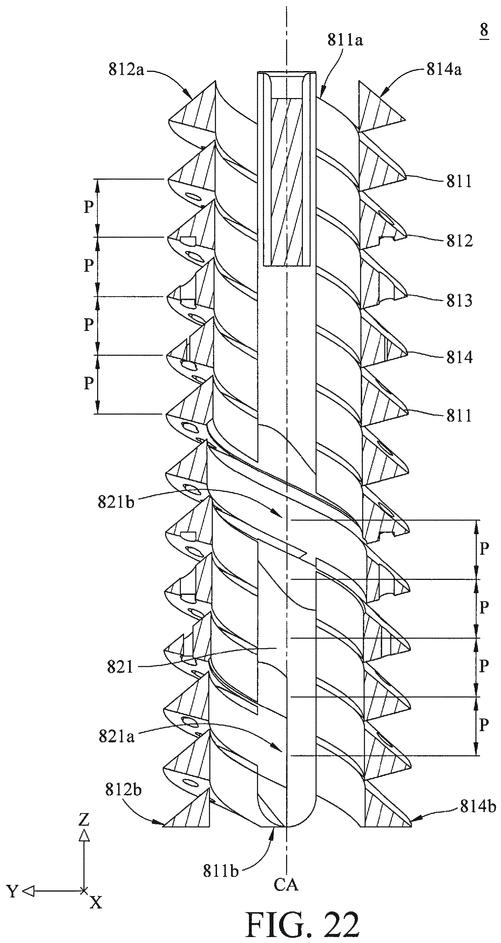

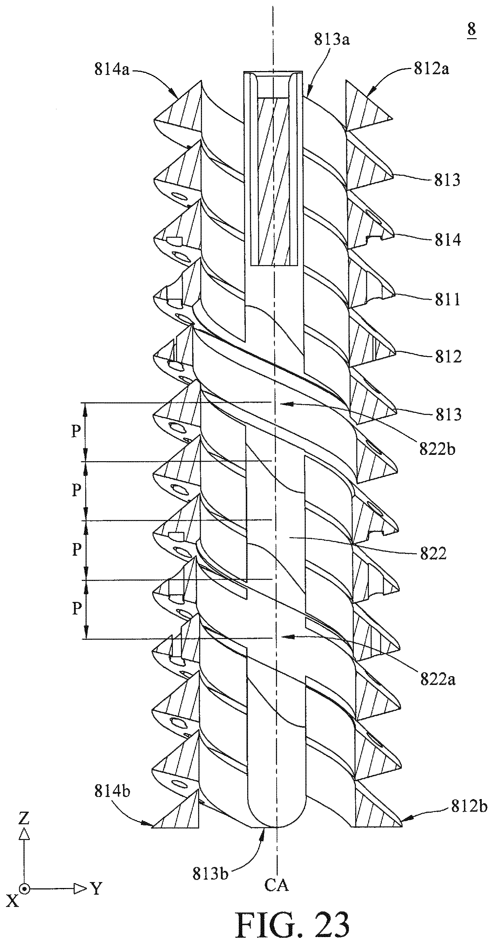

Please refer to FIG. 21 to FIG. 23. FIG. 21 is a perspective view of a bone implant according to yet a further embodiment of the disclosure. FIG. 22 is a cross-sectional view of the bone implant in FIG. 21. FIG. 23 is another cross-sectional view of the bone implant in FIG. 21. This embodiment provides a bone implant 8, and the bone implant 8 is similar to the bone implant 3 in FIG. 8.

It is noted that, in this embodiment, the bone implant 8 may have four helix bodies 811, 812, 813 and 814. In this embodiment, the end portion 811a of the helix body 811, the end portion 812a of the helix body 812, the end portion 813a of the helix body 813 and the end portion 814a of the helix body 814 surround the accommodating space S from different positions and extend with a same direction respectively. **In** detail, the helix bodies 811, 812, 813 and 814 are radially offset with an angle difference of 90 degrees from the central axis CA, and coaxial surround 3 turns about the central axis CA of the accommodating space S respectively.

A distance between two adjacent turns of two helix bodies among the helix bodies 811, 812, 813 and 814 in the direction parallel to the central axis CA is defined as a pitch P. In this embodiment, each turn of the helix body 811 corresponds to a distance in the direction parallel to the central axis CA equal to four times of pitch P, each turn of the helix body 812 corresponds to a distance in the direction parallel to the central axis CA equal to four times of pitch P, each turn of the helix body 813 corresponds to a distance in the direction parallel to the central axis CA equal to four times of pitch P and each turn of the helix body 814 corresponds to a distance in the direction parallel to the central axis CA equal to four times of pitch P.

In this embodiment, the pillar 821 is disposed in the accommodating space S and is inseparably connected to the helix bodies 811, 812, 813 and 814. The pillar 821 may have a narrowing type of notch 821a and a truncated type of notch 821b located in an order from the end portions 811b, 812b, 813b and 814b to the end portions 811a, 812a, 813a and 814a. A distance between a center of the notch 821a and a center of the notch 821b may be equal to four times of pitch P, but the present disclosure is not limited thereto.

In this embodiment, the pillar 822 is disposed in the accommodating space S and is inseparably connected to the helix bodies 811, 812, 813 and 814. The pillar 822 may have a narrowing type of notch 822a and a truncated type of notch 822b located in the order from the end portions 811b, 812b, 813b and 814b to the end portions 811a, 812a, 813a and 814a. A distance between a center of the notch 822a and a center of the notch 822b may be equal to four times of pitch P, but the present disclosure is not limited thereto.

In this embodiment, the notches 821a and 821b of the pillar 821 and the notch 822a of the pillar 822 are located closer to the end portions 811b, 812b, 813b and 814b than the end portions 811a, 812a, 813a and 814a. The notch 822b of the pillar 822 is located closer to the end portions 811a, 812a, 813a and 814a than the end portions 811b, 812b, 813b **and 814b,** but the present disclosure is not limited thereto. **In** other embodiments, the quantities and the arrangement of the notches on the pillars 821 and 822 may be modified according to the required flexibility of the bone implant 8. The flexibility of the bone implant 8 may be increased due to the narrowing type of notches and/or the truncated type of notches on the pillars 821 and/or 822.

The following introduces a plurality of bone implants similar to the bone implant 8 in FIG. 21 with unchanged parameters in the quantities of the pillars, the quantities of the notches, the pitch quantities of the distances between the notches, the quantities of the helix bodies, the outer diameters of the helix bodies, the lengths of the helix bodies, and the thread depths of the helix bodies. Compare with the maximum axial displacements of the bone implants in the central axis CA in different amount of turns. The larger displacement of the bone implant in the central axis CA, the greater flexibility. The results are shown in Table 3.

**≪Table 3≫**

| amount of turns | maximum axial displacement (mm) |
|---|---|
| 2 | 0.1079 |
| 2.25 | 0.1499 |
| 2.5 | 0.1490 |
| 2.75 | 0.1531 |
| 3 | 0.1686 |

It can be seen that the larger turns of the helix bodies of the bone implants in 2 to 3 turns make the greater maximum axial displacement and thus having greater flexibility.

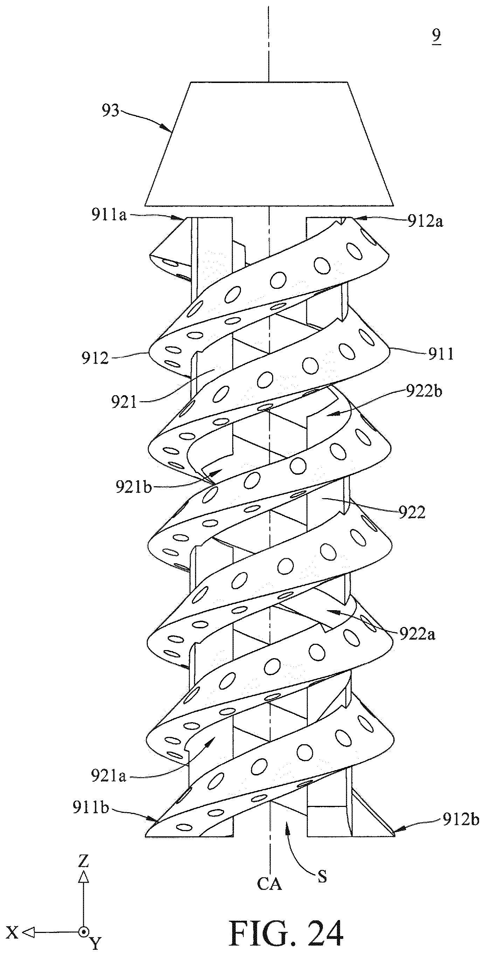

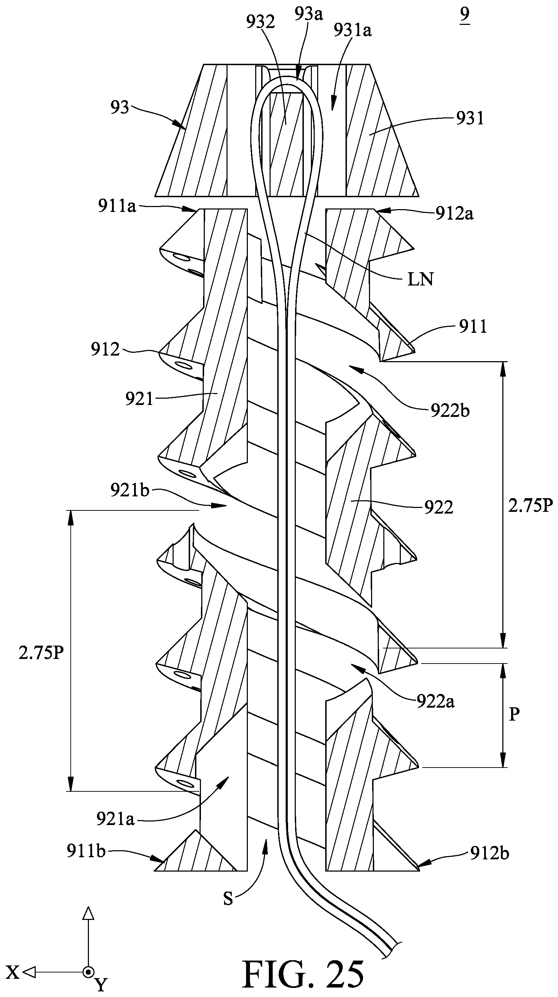

Please refer to FIG. 24 and FIG. 25. FIG. 24 is a perspective view of a bone implant according to still yet a further embodiment of the disclosure. FIG. 25 is a cross-sectional view of the bone implant in FIG. 24.

This embodiment provides a bone implant 9. The bone implant 9 may include two helix bodies 911 and 912, two pillars 921 and 922 and a holding block 93.

**In** this embodiment, the helix bodies 911 and 912 are radially offset with an angle difference of 180 degrees from a central axis CA, and coaxial surround 3 turns about the central axis CA of the accommodating space S respectively, but the present disclosure is not limited thereto. **In** some other embodiments, the quantity of the helix bodies and/or the quantity of the turns may be modified.

**In** this embodiment, the quantity of the helix bodies 911 and 912 is two, but it is not restricted.

**In** this embodiment, the pillar 921 is disposed in the accommodating space S and is inseparably connected to the helix bodies 911 and 912. The pillar 921 may have a narrowing type of notch 921a and a truncated type of notch 921b located in an order from the end portions 911b and 912b to the end portions 911a and 912a. A distance between a center of the notch 921a and a center of the notch 921b may be equal to 2.75 times of pitch P, but the present disclosure is not limited thereto.

In this embodiment, the pillar 922 is disposed in the accommodating space S and is inseparably connected to the helix bodies 911 and 912. The pillar 922 may have two truncated type of notches 921a and 921b located in the order from the end portions 911b and 912b to the end portions 911a and 912a. A distance between a center of the notch 922a and a center of the notch 922b may be equal to 2.75 times of pitch P, but the present disclosure is not limited thereto.

In this embodiment, the notch 921a of the pillar 921 and the notch 922a of the pillar 922 are located closer to the end portions 911b and 912b than the end portions 911a and 912a. The notches 921b and 922b are located closer to the end portions 911a and 912a than the end portions 911b and 912b, but the present disclosure is not limited thereto. In other embodiments, the quantities and the arrangement of the notches on the pillars 921 and 922 may be modified according to the required flexibility of the bone implant 9. The flexibility of the bone implant 9 may be increased due to the narrowing type of notches and/or the truncated type of notches on the pillars 921 and/or 922.

The holding block 93 is detachably disposed on the end portion 911a of the helix body 911 and the end portion 912a of the helix body 912. The holding block 93 includes an outer part 931 and a stopper 932. The outer part 931 may be in a cone shape and has a through hole 931a which is in a cylinder shape. A bottom surface of the outer part 931 faces the helix bodies 911 and 912. The stopper 932 is disposed in the through hole 931a and inseparably connected to the outer part 931. The stopper 932 has a recess 93a formed on a surface of the stopper 932 facing away from the helix bodies 911 and 912.

Before the bone implant 9 is implanted into bone, a suture LN can be disposed through the through hole 931a of the outer part 931 so as to be looped on the stopper 932 and located at the recess 93a of the stopper 932, such that a part of the suture LN is in the through hole 931a and the recess 93a. And then, the other part of the suture LN is disposed into the accommodating space S from the ends portions 911a and 912a to the ends portions 911b and 912b so as to be disposed through the helix bodies 911 and 912.

Then, the holding block 93 may be placed in a manmade hole on the bone, and the end portions 911a and 912a of the helix bodies 911 and 912 are then placed against the holding block 93. And then, the tool for screwing the bone implant 9 is placed into the accommodating space S to clamp the pillars 921 and 922 without interfering with the suture LN, such that the pillars 921 and 922 and the helix bodies 911 and 912 can be rotated about the central axis CA while the pillars 921 and 922 are being rotated by the tool. As a result, the helix bodies 911 and 912 are gradually screwed into the bone and thus pushing the holding block 93 deeper into the bone. Finally, the bone implant 9 is completely implanted into the bone. While implanting the bone implant 9, the holding block 93 does not rotate so that the suture LN does not rotate and will not be tangled.

According to the bone implants as discussed above, the bone implant has the pillar to maintain the overall structural strength and has the notches to improve the flexibility. As such, the bone implants of the disclosure are able to decrease the movement limitation to the injury portion and prevent itself from being detached. **In** addition, in one of the embodiments of the disclosure, the holding block may be detachable form the helix body, such that the suture will not be rotated with the helix body, thereby preventing the suture from being tangled.

It will be apparent to those skilled in the art that various modifications and variations can be made to the present disclosure. It is intended that the specification and examples be considered as exemplary embodiments only, with a scope of the disclosure being indicated by the following claims.

### [SYMBOL DESCRIPTION]

- 1, 2, 3, 4, 5, 6, 7, 8, 9: bone implant
- 11, 211, 212, 311, 312, 411, 412, 511, 512, 611: helix body
- 612, 711, 712, 713, 811, 812, 813, 814, 911, 912: helix body
- 11a, 11b, 211a, 211b, 212a, 212b, 411a: end portion
- 411b, 412a, 412b, 511a, 511b, 512a, 512b: end portion
- 611a, 611b, 612a, 612b, 711a, 711b, 712a: end portion
- 712b, 713a, 713b, 811a, 811b, 812a, 812b: end portion
- 813a, 813b, 814a, 814b, 911a, 911b, 912a, 912b: end portion
- 12, 221, 222, 421, 422, 521, 522: pillar
- 621, 622, 721, 722, 821, 822, 921, 922: pillar
- 12a, 12b, 221a, 221b, 222a, 222b, 421a: notch
- 421b, 421c, 422a, 422b, 422c, 521a, 521b: notch
- 521c, 522a, 522b, 522c, 621a, 621b, 621c: notch
- 622a, 622b, 721a, 721b, 721c, 722a, 722b: notch
- 821a, 821b, 822a, 822b, 921a, 921b, 922a, 922b: notch
- 13, 23, 93: holding block
- 13a, 23a, 93a: recess
- 31a: hole
- 641, 642: wall
- 931: outer part
- 931a: through hole
- 932: stopper
- CA: central axis
- D: outer diameter
- H: thread depth
- L: length
- LN: suture
- P: pitch
- S: accommodating space
- W1, W2: minimum width

## Claims

1. A flexible bone implant (1, 2, 3, 4, 5, 6, 7, 8, 9), comprising:
at least one helix body (11, 211, 212, 311, 312, 411, 412, 511, 512, 611, 612, 711, 712, 713, 811, 812, 813, 814, 911, 912) surrounding an accommodating space (S); and
at least one pillar (12, 221, 421, 422, 521, 621, 721, 821, 822, 921) disposed in the accommodating space (S) and inseparably connected to the at least one helix body (11, 211, 212, 311, 312, 411, 412, 511, 512, 611, 612, 711, 712, 713, 811, 812, 813, 814, 911, 912), the at least one pillar (12, 221, 421, 422, 521, 621, 721, 821, 822, 921) has a first notch (12a, 221a, 421a, 422c, 521a, 621a, 621c, 721a, 721c, 821a, 822a, 921a) and a second notch (12b, 221b, 421b, 422b, 521b, 621b, 721b, 821b, 822b, 92' the second notch (12b, 221b, 421b, 422b, 521b, 621b, 721b, 821b, 822b, 921b) divides the at least one pillar (12, 221, 421, 422, 521, 621, 721, 821, 822, 921) in parts;
**characterized in that**
the first notch narrows the at least one pillar.

2. The bone implant according to claim 1, further comprising a holding block (13, 23), wherein the holding block is inseparably disposed on at least one of the at least one pillar and the at least one helix body.

3. The bone implant according to claim 1, further comprising a holding block (93), wherein the holding block is detachably disposed on an end portion (911a, 912a) of the at least one helix body.

4. The bone implant according to claim 1, further comprising a holding block (13, 23, 93), wherein the holding block is disposed on the at least one helix body, and the holding block has a recess (13a, 23a, 93a) formed on a surface of the at least one helix body facing away from an end portion (11b, 211b, 212b, 911b, 912b) of the at least one helix body.

5. The bone implant according to claim 1, wherein the quantity of the at least one helix body is plural, the helix bodies are radially offset from a central axis (CA) of one of the helix bodies and coaxial surround about the central axis of the accommodating space.

6. The bone implant according to claim 5, the quantity of the helix bodies ranges from 2 to 4.

7. The bone implant according to claim 1, wherein the at least one helix body consists of turns ranging from 2 to 6.

8. The bone implant according to claim 1, wherein the quantity of the at least one pillar is plural, the pillars are respectively located at different sides of a central axis (CA) of the at least one helix body.

9. The bone implant according to claim 1, wherein the at least one helix body has a plurality of holes (31a).

10. The bone implant according to claim 1, further comprising at least one wall (641, 642) inseparably connected to the at least one helix body, extending from the at least one helix body in a central axis (CA) and surrounding the accommodating space.

11. The bone implant according to claim 10, wherein the at least one wall has a plurality of holes.

12. The bone implant according to claim 1, wherein the at least one helix body has an outer diameter (D) and a length (L) in a central axis (CA) of the accommodating space, and the outer diameter and the length have a ratio between 1 and 1/15.

13. The bone implant according to claim 1, wherein the accommodating space has a minimum width (W1, W2) in a radial direction passing through a central axis (CA) of the accommodating space, and the minimum width is more than 0.9 mm.

14. The bone implant according to claim 1, wherein the at least one helix body has a plurality of pitches (P), and the at least one pillar has the at least one notch in every one to four pitches.

## Patentansprüche

1. Ein flexibles Knochenimplantat (1, 2, 3, 4, 5, 6, 7, 8, 9), umfassend:
mindestens einen Helixkörper (11, 211, 212, 311, 312, 411, 412, 511, 512, 611, 612, 711, 712, 713, 811, 812, 813, 814, 911, 912), der einen Aufnahmeraum (S) umgibt; und
mindestens eine Säule (12, 221, 421, 422, 521, 621, 721, 821, 822, 921), die in dem Aufnahmeraum (S) angeordnet und untrennbar mit dem mindestens einen Helixkörper (11, 211, 212, 311, 312, 411, 412, 511, 512, 611, 612, 711, 712, 713, 811, 812, 813, 814, 911, 912) verbunden ist,
die mindestens eine Säule (12, 221, 421, 422, 521, 621, 721, 821, 822, 921) hat eine erste Kerbe (12a, 221a, 421a, 422c, 521a, 621a, 621c, 721a, 721c, 821a, 822a, 921a) und eine zweite Kerbe (12b, 221b, 421b, 422b, 521b, 621b, 721b, 821b, 822b, 921b),
die zweite Kerbe (12b, 221b, 421b, 422b, 521b, 621b, 721b, 821b, 822b, 921b) teilt die mindestens eine Säule (12, 221, 421, 422, 521, 621, 721, 821, 822, 921) in Teile;
**dadurch gekennzeichnet, dass** die erste Kerbe die mindestens eine Säule verschmälert.

2. Das Knochenimplantat nach Anspruch 1, ferner umfassend einen Halteblock (13, 23), wobei der Halteblock untrennbar an mindestens einer der mindestens einen Säule und dem mindestens einen Helixkörper angeordnet ist.

3. Das Knochenimplantat nach Anspruch 1, ferner umfassend einen Halteblock (93), wobei der Halteblock abnehmbar an einem Endabschnitt (911a, 912a) des mindestens einen Helixkörpers angeordnet ist.

4. Das Knochenimplantat nach Anspruch 1, ferner umfassend einen Halteblock (13, 23, 93), wobei der Halteblock auf dem mindestens einen Helixkörper angeordnet ist und der Halteblock eine Aussparung (13a, 23a, 93a) aufweist, die auf einer Oberfläche des mindestens einen Helixkörpers ausgebildet ist, die von einem Endabschnitt (11b, 211b, 212b, 911b, 912b) des mindestens einen Helixkörpers weg weist.

5. Das Knochenimplantat nach Anspruch 1, wobei die Anzahl des mindestens einen Helixkörpers eine Mehrzahl ist, die Helixkörper radial von einer zentralen Achse (CA) eines der Helixkörper versetzt sind und koaxial um die zentrale Achse des Aufnahmeraums umgeben.

6. Das Knochenimplantat nach Anspruch 5, wobei die Anzahl der Helixkörper zwischen 2 und 4 liegt.

7. Das Knochenimplantat nach Anspruch 1, wobei der mindestens eine Helixkörper aus Windungen im Bereich von 2 bis 6 besteht.

8. Das Knochenimplantat nach Anspruch 1, wobei die Anzahl der mindestens einen Säule eine Mehrzahl ist und die Säulen jeweils auf verschiedenen Seiten einer zentralen Achse (CA) des mindestens einen Helixkörpers angeordnet sind.

9. Das Knochenimplantat nach Anspruch 1, wobei der mindestens eine Helixkörper eine Vielzahl von Löchern (31a) aufweist.

10. Das Knochenimplantat nach Anspruch 1 umfasst ferner mindestens eine Wand (641, 642), die untrennbar mit dem mindestens einen Helixkörper verbunden ist, sich von dem mindestens einen Helixkörper in einer zentralen Achse (CA) erstreckt und den Aufnahmeraum umgibt.

11. Das Knochenimplantat nach Anspruch 10, wobei die mindestens eine Wand eine Vielzahl von Löchern aufweist.

12. Das Knochenimplantat nach Anspruch 1, wobei der mindestens eine Helixkörper einen Außendurchmesser (D) und eine Länge (L) in einer zentralen Achse (CA) des Aufnahmeraums aufweist und der Außendurchmesser und die Länge ein Verhältnis zwischen 1 und 1/15 haben.

13. Das Knochenimplantat nach Anspruch 1, wobei der Aufnahmeraum eine minimale Breite (W1, W2) in einer radialen Richtung, die durch eine zentrale Achse (CA) des Aufnahmeraums verläuft, aufweist und die minimale Breite mehr als 0,9 mm beträgt.

14. Das Knochenimplantat nach Anspruch 1, wobei der mindestens eine Helixkörper eine Vielzahl von Steigungen (P) aufweist und die mindestens eine Säule die mindestens eine Kerbe in jeder der ein bis vier Steigungen aufweist.

## Revendications

1. Implant osseux flexible (1, 2, 3, 4, 5, 6, 7, 8, 9), comprenant :
au moins un corps hélicoïdal (11, 211, 212, 311, 312, 411, 412, 511, 512, 611, 612, 711, 712, 713, 811, 812, 813, 814, 911, 912) entourant un espace de logement (S) ; et
au moins un pilier (12, 221, 421, 422, 521, 621, 721, 821, 822, 921) disposé dans l'espace de logement (S) et connecté de manière inséparable audit au moins un corps hélicoïdal (11, 211, 212, 311, 312, 411, 412, 511, 512, 611, 612, 711, 712, 713, 811, 812, 813, 814, 911, 912),
ledit au moins un pilier (12, 221, 421, 422, 521, 621, 721, 821, 822, 921) comportant une première encoche (12a, 221a, 421a, 422c, 521a, 621a, 621c, 721a, 721c, 821a, 822a, 921a) et une deuxième encoche (12b, 221b, 421b, 422b, 521b, 621b, 721b, 821b, 822b, 921b), la deuxième encoche (12b, 221b, 421b, 422b, 521b, 621b, 721b, 821b, 822b, 921b) divisant ledit au moins un pilier (12, 221, 421, 422, 521, 621, 721, 821, 822, 921) en parties ;
**caractérisé en ce que**
la première encoche rétrécit ledit au moins un pilier.

2. Implant osseux selon la revendication 1, comprenant en outre un bloc de maintien (13, 23), dans lequel le bloc de maintien est disposé de manière inséparable sur au moins un élément parmi ledit au moins un pilier et ledit au moins un corps hélicoïdal.

3. Implant osseux selon la revendication 1, comprenant en outre un bloc de maintien (93), dans lequel le bloc de maintien est disposé de manière détachable sur une portion d'extrémité (911a, 912a) dudit au moins un corps hélicoïdal.

4. Implant osseux selon la revendication 1, comprenant en outre un bloc de maintien (13, 23, 93), dans lequel le bloc de maintien est disposé sur ledit au moins un corps hélicoïdal, et le bloc de maintien comporte un évidement (13a, 23a, 93a) formé sur une surface dudit au moins un corps hélicoïdal opposée à une portion d'extrémité (11b, 211b, 212b, 911b, 912b) dudit au moins un corps hélicoïdal.

5. Implant osseux selon la revendication 1, dans lequel la quantité dudit au moins un corps hélicoïdal est plurielle, les corps hélicoïdaux étant décalés radialement par rapport à un axe central (CA) de l'un des corps hélicoïdaux et entourant coaxialement l'axe central de l'espace de logement.

6. Implant osseux selon la revendication 5, dans lequel la quantité des corps hélicoïdaux est comprise entre 2 et 4.

7. Implant osseux selon la revendication 1, dans lequel ledit au moins un corps hélicoïdal comporte entre 2 et 6 spires.

8. Implant osseux selon la revendication 1, dans lequel la quantité dudit au moins un pilier est plurielle, les piliers étant respectivement situés sur différents côtés d'un axe central (CA) dudit au moins un corps hélicoïdal.

9. Implant osseux selon la revendication 1, dans lequel ledit au moins un corps hélicoïdal comporte une pluralité de trous (31a).

10. Implant osseux selon la revendication 1, comprenant en outre au moins une paroi (641, 642) connectée de manière inséparable audit au moins un corps hélicoïdal, et qui s'étend depuis ledit au moins un corps hélicoïdal dans un axe central (CA) et entoure l'espace de logement.

11. Implant osseux selon la revendication 10, dans lequel ladite au moins une paroi comporte une pluralité de trous.

12. Implant osseux selon la revendication 1, dans lequel ledit au moins un corps hélicoïdal présente un diamètre externe (D) et une longueur (L) dans un axe central (CA) de l'espace de logement, et le diamètre externe et la longueur présentent un ratio compris entre 1 et 1/15.

13. Implant osseux selon la revendication 1, dans lequel l'espace de logement présente une largeur minimale (W1, W2) dans une direction radiale passant par un axe central (CA) de l'espace de logement, et la largeur minimale est supérieure à 0,9 mm.

14. Implant osseux selon la revendication 1, dans lequel ledit au moins un corps hélicoïdal comporte une pluralité de pas (P), et ledit au moins un pilier comporte ladite au moins une encoche tous les un à quatre pas.
